# EUROPEAN PATENT SPECIFICATION

(11) **EP 3 752 826 B1**
(45) Date of publication and mention of the grant of the patent: **28.10.2026**
(21) Application number: 19708724.0
(22) Date of filing: 14.02.2019
(51) Int. Cl.: G01N 30/88

(54) **METHOD FOR FAILURE MODE DETECTION IN PROCESS CHROMATOGRAPHY**
VERFAHREN ZUR FEHLERERKENNUNG IN DER PROZESSCHROMATOGRAFIE
PROCÉDÉ DE DÉTECTION DE MODE DE DÉFAILLANCE DANS UNE CHROMATOGRAPHIE DE TRAITEMENT

(30) Priority: 15.02.2018 US 201862631167 P
(43) Date of publication of application: 23.12.2020
(73) Proprietor: Regeneron Pharmaceuticals, Inc., Tarrytown, NY 10591 (US)
(72) Inventor: MAO, Nathan L., New York 10591 (US); CARVER, Scott, New York 10591 (US); SCHILLING, Bernhard, New York 10591 (US); SHIERLY, Eric, New York 10591 (US)
(74) Representative: Carpmaels & Ransford LLP
(86) International application number: PCT/US2019/017942
(87) International publication number: WO 2019/161012

(56) References cited:
- WO-A2-2009/094203
- JP-A- 2006 292 446
- US-A- 4 802 981
- US-A1- 2010 064 770
- US-B2- 7 732 216

## Description

### RELATED APPLICATIONS

This application claims the benefit of USSN 62/631,167, filed on February 15, 2018.

### FIELD OF THE INVENTION

The present disclosure relates generally to purification methods for biologic products, including the real-time detection of errors in a chromatography process.

### BACKGROUND OF THE INVENTION

A successful timely identification of failure mode in a protein chromatography operation can be challenging not only because the failure mode is specific to each unit operation associated with a program but also because an automatic detection method is not readily available. This is particularly true with large-scale manufacturing. Thus, there is a long-felt yet unmet need for a system and method of in-process, scalable, failure mode detection. The disclosure provides such a novel method to address and solve this need.
US7732216B2 describes methods for testing the chromatography type and/or the integrity of chromatography membranes or monoliths, sealed in a housing.

### SUMMARY OF THE INVENTION

The disclosure provides a method for monitoring process chromatograms in an automated or semi-automated fashion. In some embodiments of the methods of the disclosure, a measured absorbance intensity within an integral volume window is compared to a maximum absorbance intensity inferred from at least one reference chromatogram, this measured absorbance intensity is used to identify instability in the chromatography matrix, and by predicting and/or identifying an impending failure, the method improves process performance on any scale. Accordingly, the present disclosure provides a method useful for failure mode detection in a chromatography process.

The disclosure provides a method as claimed for predicting or detecting a malfunction in a chromatography process, comprising: (a) generating a reference chromatogram from a chromatography column, (b) generating a process chromatogram from the chromatography column, and (c) detecting an atypical profile in the process chromatogram, wherein the atypical profile indicates a potential or actual malfunction of a chromatography process.

The method further comprises (d) generating a warning signal.

In some embodiments of the methods of the disclosure, the method further (e) repacking the chromatography column.

In some embodiments of the methods of the disclosure, the chromatography column is suitable for use in ion exchange chromatography.

In some embodiments of the methods of the disclosure, the chromatography column is suitable for use in affinity chromatography.

In some embodiments of the methods of the disclosure, the chromatography column is suitable for use in hydrophobic interaction chromatography.

In some embodiments of the methods of the disclosure, the potential or actual malfunction of the chromatography process is caused by column deterioration. In some embodiments, the column deterioration comprises column bed fracturing.

In some embodiments of the methods of the disclosure, the atypical profile comprises an additional peak on the process chromatogram when compared to a comparable position on the reference chromatogram. The additional peak occurs during a washing step and precedes the elution step in the chromatography process.

In some embodiments of the methods of the disclosure, including those wherein the atypical profile comprises an additional peak on the process chromatogram when compared to a comparable position on the reference chromatogram, the magnitude of the additional peak indicates a degree of severity of the potential or actual malfunction of the chromatography process. In some embodiments, an increase in the magnitude of the additional peak indicates an increase in the degree of severity of the potential or actual malfunction of the chromatography process. In some embodiments, the malfunction of the chromatography process induces a loss of separation power of the chromatography column.

In some embodiments of the methods of the disclosure, including those wherein the atypical profile comprises an additional peak on the process chromatogram when compared to a comparable position on the reference chromatogram, the atypical profile comprises at least one peak or at least one indentation on the process chromatogram having a decreased sharpness or a decreased magnitude when compared to a peak or an indentation at a comparable position on the reference chromatogram. In some embodiments, the at least one peak or the at least one indentation on the process chromatogram having a decreased sharpness or a decreased magnitude occurs during the washing step. In some embodiments, the at least one peak or the at least one indentation on the process chromatogram having a decreased sharpness or a decreased magnitude follows the elution step.

In some embodiments of the methods of the disclosure, including those wherein the atypical profile comprises an additional peak on the process chromatogram when compared to a comparable position on the reference chromatogram, the atypical profile comprises a first peak or a first indentation having a decreased sharpness or a decreased magnitude on the process chromatogram when compared to a peak or an indentation at a comparable first position on the reference chromatogram and a second peak or a second indentation having a decreased sharpness or a decreased magnitude on the process chromatogram when compared to a peak or indentation at a comparable second position on the reference chromatogram. In some embodiments, the first peak or the first indentation follows the elution step. In some embodiments, the first peak or the first indentation corresponds to an indentation occurring at a start of a collecting step. In some embodiments, the second peak or the second indentation follows a collection step. In some embodiments, the second peak or the second indentation comprises a strip peak.

The process chromatogram comprises a measured absorbance intensity, a maximum absorbance intensity, and an integral volume window. In some embodiments, the atypical profile comprises a measured absorbance intensity that exceeds a maximum absorbance intensity during an integral volume window. In some embodiments, the measured absorbance intensity is determined at a wavelength corresponding to ultraviolet (UV) light. In some embodiments, the measured absorbance intensity is determined at a wavelength of between 260 nanometers (nm) and 280 nm, inclusive of the endpoints. In some embodiments, the measured absorbance intensity is determined at a wavelength of 260 nm, providing an A₂₆₀ value. In some embodiments, the measured absorbance intensity is determined at a wavelength of 280 nm, providing an A₂₈₀ value. In some embodiments, the atypical profile comprises a measured absorbance intensity that exceeds a maximum absorbance intensity during an integral volume window and wherein the maximum absorbance intensity has an A₂₆₀ value or an A₂₈₀ value of at least 0.05. In some embodiments of the methods of the disclosure, a typical profile comprises a measured absorbance intensity that does not exceed a maximum absorbance intensity during an integral volume window and wherein the maximum absorbance intensity has an A₂₆₀ value or an A₂₈₀ value of 0.05 or less.

In some embodiments of the methods of the disclosure, a value of an absorbance intensity of an atypical profile and/or a typical profile is measured during an elution step of a preceding column process. In some embodiments, a value of an absorbance intensity of an atypical profile and/or a typical profile is determined at a wavelength corresponding to ultraviolet (UV) light. In some embodiments, the measured absorbance intensity is determined at a wavelength of between 260 nanometers (nm) and 280 nm, inclusive of the endpoints. In some embodiments, the measured absorbance intensity is determined at a wavelength of 260 nm, providing an A₂₆₀ value. In some embodiments, the measured absorbance intensity is determined at a wavelength of 280 nm, providing an A₂₈₀ value. In some embodiments, a signal is sent following a value indicating an atypical profile, which indicates column deterioration.

In some embodiments of the methods of the disclosure, a value of an absorbance intensity of an atypical profile and/or a typical profile is measured during an elution step of a preceding column process. In some embodiments, a value of an absorbance intensity of an atypical profile and/or a typical profile is determined by assessing a pH level of an eluate during an elution step. In some embodiments, the pH indicated an atypical profile because the pH value is higher than expected from a typical profile. In some embodiments, the pH indicated an atypical profile because the pH value is lower than expected from a typical profile. In some embodiments, a signal is sent following a value indicating an atypical profile, which indicates column deterioration.

In some embodiments of the methods of the disclosure, the integral volume window occurs between 1 L and 5,000 L, inclusive of the endpoints. In some embodiments of the methods of the disclosure, the integral volume window occurs between 1000 L and 3,000 L, inclusive of the endpoints. In some embodiments of the methods of the disclosure, the integral volume window occurs between 1000 L and 2500 L, inclusive of the endpoints. In some embodiments, the integral volume window occurs between 1250 L and 2250 L, inclusive of the endpoints. In some embodiments, the integral volume window occurs between 1600 L and 1800 L, inclusive of the endpoints. In some embodiments of the methods of the disclosure, the integral volume window occurs over a volume of 1L, 10L, 100L, 250L, 500L, 1000L, 1500L, 2000L, 2500L, 3000L, 4000L, 5000L or any number of liters in between.

In some embodiments of the methods of the disclosure, the chromatography column comprises a Q Sepharose anion exchange resin.

In some embodiments of the methods of the disclosure, the step of (a) generating the reference chromatogram comprises (1) contacting a starting composition comprising a plurality of pre-determined analytes and a separation composition comprising a matrix, under conditions sufficient for at least one analyte of the starting composition to reversibly bind to the matrix, wherein the chromatography column comprises the separation composition, to produce an analyte-bound column and a first waste composition; (2) contacting the analyte-bound column of (1) and a washing composition, wherein only the at least one analyte of the starting composition remains bound to the matrix to produce a purified analyte-bound column and a second waste composition; (3) contacting the purified analyte-bound column of (2) and an elution composition, wherein the at least one analyte of the starting composition is released from the matrix into the elution composition to produce an eluted analyte composition; (4) collecting the eluted analyte composition; (5) simultaneously with each of steps (1)-(4), contacting each of the first waste composition, the second waste composition, and the eluted analyte composition with ultraviolet (UV) light, (6) measuring an absorbance of the UV light of each of the first waste composition, the second waste composition, and the eluted analyte composition to produce an absorbance value for each of the first waste composition, the second waste composition, and the eluted analyte composition; and (7) generating a chromatogram by plotting the absorbance value of each of the first waste composition, the second waste composition, and the eluted analyte composition as a function of volume of liquid that traverses the chromatography column.

In some embodiments of the methods of the disclosure, the step of (b) generating the process chromatogram comprises (1) contacting a starting composition comprising a plurality of test analytes and a separation composition comprising a matrix, under conditions sufficient for at least one analyte of the starting composition to reversibly bind to the matrix, wherein the chromatography column comprises the separation composition, to produce an analyte-bound column and a first waste composition; (2) contacting the analyte-bound column of (1) and a washing composition, wherein only the at least one analyte of the starting composition remains bound to the matrix to produce a purified analyte-bound column and a second waste composition; (3) contacting the purified analyte-bound column of (2) and an elution composition, wherein the at least one analyte of the starting composition is released from the matrix into the elution composition to produce an eluted analyte composition; (4) collecting the eluted analyte composition; (5) simultaneously with each of steps (1)-(4), contacting each of the first waste composition, the second waste composition, and the eluted analyte composition with ultraviolet (UV) light, (6) measuring an absorbance of the UV light of each of the first waste composition, the second waste composition, and the eluted analyte composition to produce an absorbance value for each of the first waste composition, the second waste composition, and the eluted analyte composition; and (7) generating a chromatogram by plotting the absorbance value of each of the first waste composition, the second waste composition, and the eluted analyte composition as a function of volume of liquid that traverses the chromatography column.

In some embodiments of the methods of the disclosure, steps (a) or (b) generating a chromatogram comprise(s) a contacting step (5) and a measuring step (6), and furthermore, the contacting step (5) and measuring step (6) are continuous. In some embodiments, the contacting step (5) and measuring step (6) are simultaneous, sequential or alternating, and, continuous.

In some embodiments of the methods of the disclosure, the chromatography column comprises a matrix and the matrix comprises a resin.

In some embodiments of the methods of the disclosure, and in particular, with reference to steps (a) or (b) generating a chromatogram, test analyte is a biologic composition. In some embodiments, the biologic composition is a therapeutic composition for use in a method of treatment of a human subject. In some embodiments, the biologic composition comprises a therapeutic protein.

In some embodiments of the methods of the disclosure, the warning signal is communicated electronically to a processor and the processor is operably linked to the chromatography column. In some embodiments, the processor receives the warning signal and interrupts the chromatography process or prevents the initiation of a further chromatography process until the potential or actual malfunction of the chromatography column is resolved.

Additional aspects and embodiments of the invention will be apparent from the detailed description that follows.

### BRIEF DESCRIPTION OF THE DRAWINGS

FIGURE 1 represents the reference chromatograms, that is the historical norm.
FIGURE 2 represents another reference chromatogram.
FIGURE 3 represents a chromatogram displaying an irregular Wash 2 peak.
FIGURE 4 represent a chromatogram displaying an irregular Wash 2 peak, irregular elution peaks, and loss of sharpness in the Strip peak.
FIGURE 5 represents a failure chromatogram. The Wash 2 peak is present.
FIGURE 6 represents comparison of typical and atypical VEGF-Trap Q chromatograms. The majority of atypical UV traces have an absorbance ≥ 0.05 au at 635 liters into Wash 2.
FIGURE 7 represents additional atypical VEGF-Trap Q chromatograms, that is, Wash 2 peak examples.
FIGURE 8 represents additional typical VEGF-Trap Q chromatograms. UV traces do not exceed an absorbance of 0.05 at 635 liters into Wash 2.

### DETAILED DESCRIPTION

A successful timely identification of failure mode in protein chromatography operation, especially in large-scale manufacturing, can be challenging not only because the failure mode is specific to each unit operation associated with a program but also, in the absence of the compositions and methods of the disclosure, an automatic detection method is not readily available.

The disclosure provides a novel Watch system and method for failure mode detection. This new Watch system and method of chromatography column monitoring is intended to alert an operator if the column is not performing its intended function. The Watch systems and methods of the disclosure automatically monitors system signals at integral times to determine if the column is performing optimally. If the system fails to meet the acceptance criteria, a message is displayed to the operator instructing them to add the event to the quality management system and to allow for further investigation and potentially remediation. Monitored signals, significant signal intensity, and integral time windows are all empirically determined for each unique chromatography unit operation to be monitored.

### Chromatography

The systems and methods of the disclosure may be used for preparative or analytical chromatography. In some embodiments, methods of the disclosure are used to purify proteins that may be used in a composition for the treatment of a disease. Especially for those methods involving preparatory chromatography for the large scale or high volume purification of a therapeutic protein, efficiency of the purification process is essential. The systems and methods of the disclosure may be used at any scale. However, they are particularly useful when a large number of preparations are in process and when a malfunctioning system (such as a chromatography column that is allowing valuable analyte to pass though the separation composition when it should be capturing it), results in loss of a valuable product and valuable production time. The systems and methods of the disclosure predict and detect a malfunction in a chromatography process in real-time and, as a result of having identified the malfunctioning process, enable the restoration of function and return to efficient and reliable purification before additional work is performed using the malfunctioning process.

The disclosure encompasses any form of chromatography. As a technique, chromatography is a method of separating components from a mixture. Separation is achieved though the portioning of a mobile phase and a stationary phase. As described herein, the starting compositions comprising a test analyte, optionally also for preparation, belong to the mobile phase. As described herein, separation compositions comprising a matrix belong to the stationary phase. Exemplary matrices of the disclosure may comprise any material having the ability to separate elements of the mobile phase based on chemical properties (charge) and/or physical properties (size).

As used in some embodiments of the disclosure, including methods of preparation of therapeutic proteins, chromatography processes may include, but are not limited to, column and planar chromatography. In some preferred embodiments, chromatography processes may include, but are not limited to, column chromatography.

### Column Chromatography

The disclosure encompasses any form of column chromatography, including wet and dry methods. In a dry method, the separation composition comprises a dry matrix, which is subsequently contacted with a liquid starting composition, optimally, wetting the entire matrix and not permitting the matrix to dry until the end of the entire process. In a wet method, the separation composition comprises suspension or slurry of a matrix and an eluent, which is introduced into the column and, subsequently contacted with a liquid starting composition.

### Ion Exchange Chromatography

In some embodiments of the disclosure, the separation composition may comprise any material having the ability to separate elements of the mobile phase based on charge. For example, the separation composition may comprise a matrix that reversibly binds a charged protein analyte from a starting composition. Thus, the separation matrix may be suitable for anion- or cation-exchange chromatography and may comprise a matrix having net negative or positive charge, respectively. In some embodiments of the disclosure, the separation composition comprises a matrix comprising sepharose or Q sepharose. In some embodiments of the disclosure, the chromatography column is a Q column.

### Affinity Chromatography

In some embodiments of the disclosure, the separation composition may comprise any material having the ability to separate elements of the mobile phase based on specific interactions between binding partners including, but not limited to, antigen/antibody binding partners, enzyme/substrate binding partners, receptor/ligand binding partners, protein/protein binding partners, protein/nucleic acid binding partners, and nucleic acid/nucleic acid binding partners. For example, the separation composition may comprise a matrix that reversibly binds one partner of a binding partner (e.g. an analyte from a starting composition) by comprising the other partner of a binding partner. By way of example, the separation composition may comprise a matrix that comprises an antigen to which the analyte of the starting composition specifically binds to selectively purify the antibody analyte from the starting material. By way of further illustration, the separation composition may comprise a matrix that comprises an epitope of a VEGF protein to which a VEGF-Trap selectively binds to selectively purify the VEGF-Trap analyte from the starting material.

### Hydrophobic Interaction Chromatography (HIC)

In some embodiments of the disclosure, the separation composition may comprise any material having the ability to separate elements of the mobile phase based on their relative hydrophobicity. HIC may be used to selectively purify a protein analyte while maintaining its biological activity as a result of the use of conditions and of matrices that operate under less denaturing conditions. In some embodiments, analytes of the disclosure containing hydrophobic and hydrophilic regions are contacted to an HIC column in a high-salt buffer. The salt in the buffer reduces the solvation of analyte solutes in the starting material. As solvation decreases, hydrophobic regions that become exposed are adsorbed by the media. The more hydrophobic the molecule in the starting material, the less salt is needed to promote binding. A decreasing salt gradient may be used to elute samples from the column in order of increasing hydrophobicity. Elution buffers of the disclosuremay also comprise a mild organic modifier or a mild detergent.

### Size Exclusion Chromatography (SEC)

In some embodiments of the disclosure, the separation composition may comprise any material having the ability to separate elements of the mobile phase based on their relative size, or in some cases, relative molecular weights. SEC may be used to selectively purify large molecules or macromolecular complexes such as proteins and polymers. In some embodiments, for example those embodiments wherein an aqueous solution is used to transport the mobile phase through the column, the technique is known as gel-filtration chromatography. In some embodiments, for example those embodiments wherein an organic solvent is used as a mobile phase through the column, the technique is known as gel permeation chromatography. In some embodiments, the chromatography column is packed with fine, porous beads which are composed of, for example, dextran polymers (Sephadex), agarose (Sepharose), or polyacrylamide (Sephacryl or BioGel P). The pore sizes of these beads are used to estimate the dimensions of macromolecules. Gel-filtration chromatography can be used to fractionate protein analytes and other water-soluble polymers, while gel permeation chromatography can be used to fractionate organic-soluble polymers based on molecular weight distribution.

### High Performance Liquid Chromatography (HPLC)

In some embodiments of the disclosure, the separation composition may comprise any material having the ability to separate elements of the mobile phase based on their interaction with the adsorbent material of the separation composition, which causes different flow rates for the different components and leads to the separation of the components as they flow out of the column. Analytes in the mobile phase can be separated, for example, through hydrophobic, ionic or dipole-dipole interactions. HPLC relies on pumps to pass a pressurized liquid solvent containing the mobile phase through a column filled with a solid adsorbent material. In some embodiments, the active component of the column, the adsorbent comprises a granular material made of solid particles (for example, silica or polymers). Exemplary particle sizes include 2-50 micron particles, which small size can give HPLC chromatography superior resolving power. In some embodiments, the pressurized mobile phase is a mixture of solvents, such as water, acetonitrile and/or methanol. In some embodiments, an HPLC pump mixes multiple solvents together in ratios changing in time, generating a gradient composition in the mobile phase. In some embodiments, the aqueous component of the mobile phase contains acids (such as formic, phosphoric or trifluoroacetic acid) or salts to assist in the separation of the sample components. In some embodiments, the HPLC comprises partition chromatography, normal-phase chromatography, displacement chromatography, reversed-phase chromatography, size exclusion chromatography, ion-exchange chromatography, affinity chromatography, hydropobic interaction chromatography or aqueous normal-phase chromatography.

### Fast Protein Liquid Chromatography (FPLC)

In some embodiments of the disclosure, the separation composition may comprise any material having the ability to separate elements of the mobile phase based on their different affinities for the separation composition. Fast protein liquid chromatography (FPLC) is a form of liquid chromatography that is used to separate mixtures of protein analytes. In some embodiments, separation is possible because the different components of a mobile phase have different affinities for mobile phase and the stationary phase (the separation composition). In some embodiments, the mobile phase is an aqueous solution, or buffer. The buffer flow rate can be controlled by a positive-displacement pump. In some embodiments, the buffer flow rate is constant, and the composition of the buffer is varied. In some embodiments, the stationary phase is a resin composed of beads, for example agarose beads. The person of ordinary skill will be able to vary bead sizes and surface ligands of the stationary phase depending upon the mobile phase and analytes of a particular application.

In some embodiments, the FPLC comprises ion exchange chromatography. For example, a stationary phase resin binds a protein analyte by a charge interaction while in a first buffer (the running buffer), but dissociates in a second buffer (the elution buffer). A mixture containing one or more protein analytes is dissolved in the first buffer and pumped into the column. The proteins of interest bind to the resin while other components are carried out in the first buffer. The total flow rate of the buffer is kept constant. Over time, the percentage of the elution buffer is gradually increased from 0% to 100% according to a programmed change in concentration to produce a gradient. At some point along the gradient each of the bound protein analytes dissociates and appears in the eluate. The eluate then passes through detectors. Exemplary detectors can measure salt concentration of the eluate (by conductivity) and protein concentration of the eluate (by absorption of ultraviolet light).

### Pressurization

In some embodiments of the methods of the disclosure, flow of the mobile phase through the stationary phase is controlled by gravity. In some embodiments, gravity chromatography is used for single-step purifications like desalting and affinity applications. A typical gravity column comprises a top buffer reservoir element operably linked to a chromatography column with an outlet at the bottom. Flow is controlled using a stopcock or a tubing pincher between the buffer reservoir and the column. Flow adaptors can be used to minimize variability in sample loading. In some embodiments, the gravity chromatography comprises partition chromatography, normal-phase chromatography, displacement chromatography, reversed-phase chromatography, size exclusion chromatography, ion-exchange chromatography, affinity chromatography, hydropobic interaction chromatography or aqueous normal-phase chromatography.

In some embodiments of the methods of the disclosure, the chromatography comprises low pressure chromatography. In some embodiments, a low pressure chromatography system is a system that operates at pressures of less than 50 psi (~3 bar). Low-pressure chromatography can be used protein separations that do not require high resolution.

In some embodiments of the methods of the disclosure, the chromatography comprises medium pressure chromatography. In some embodiments, a medium pressure chromatography system is a system that operates at pressures of up to 3,500 psi (24 Mpa). Medium-pressure chromatography systems produce enough pressure to accommodate higher resolution stationary phase compositions, for example using 5-15 µm beads. In some embodiments, the medium pressure chromatography comprises partition chromatography, normal-phase chromatography, displacement chromatography, reversed-phase chromatography, size exclusion chromatography, ion-exchange chromatography, affinity chromatography, hydropobic interaction chromatography or aqueous normal-phase chromatography.

In some embodiments of the methods of the disclosure, the chromatography comprises high pressure chromatography, for example HPLC.

### Mobile Phase

The disclosure encompasses any form of mobile phase, including, but not limited to, an eluent, a solvent, a sterile solvent, and a pharmaceutically-acceptable carrier. Elution compositions of the disclosure may comprise a pharmaceutically-acceptable carrier.

### Stationary Phase

The disclosure encompasses any form of stationary phase, including, but not limited to, silica, alumina (Al₂O₃), cellulose, sepharose and Q sepharose. Exemplary separation compositions of the disclosure may comprise, a matrix, a polymer, a resin, a protein, or a combination thereof. In some embodiments, the separation composition comprises sepharose or Q sepharose.

### Chromatograms

As used herein, a chromatogram is the visual output of a chromatograph. Typically, retention time and/or outflow volume of the eluate are represented on the x axis of the chromatogram, and a signal is plotted on the y axis. Exemplary signals include, but are not limited to electrical conductance, light absorbance and mass spectrometry, and correspond to a response of a detector to analytes exiting the chromatograph. In some embodiments, this signal is proportional to the concentration of a specific analyte being separated by the chromatograph.

In one exemplary embodiment, protein concentration, as measured by light absorption, is plotted on the y axis of the chromatogram. Many proteins in solution absorb light, for example ultraviolet light at a wavelength of 280 nm, which can be used to calculate protein concentration in a sample. Depending on the protein, additional wavelengths of light are contemplated. For example, proteins comprising heme groups or fluorophores will be detectable with different wavelengths of light. The person of ordinary skill will be able to select a suitable detection system for a specific analyte.

### Biological Samples

In some embodiments of the methods of the disclosure, the analyte comprises a protein, a peptide, a nucleic acid or a virus. In some embodiments, the nucleic acid is deoxyribonucleic acid (DNA) or ribonucleic acid (RNA). In some embodiments, the virus is an adenovirus, an adeno-associated virus or a lentivirus.

### EXAMPLES

### Example 1: Purification of a VEGF Trap

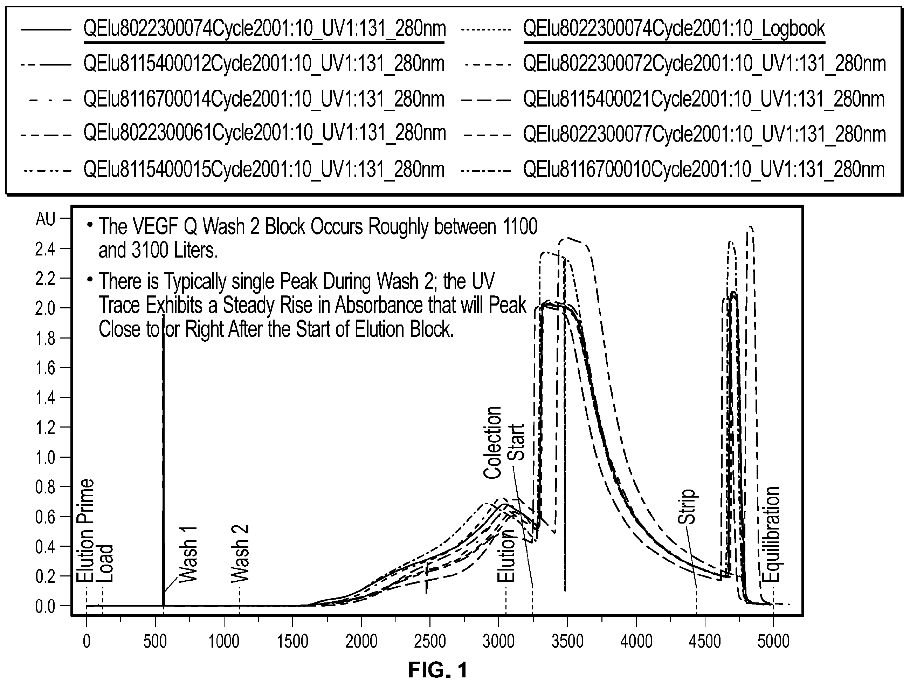

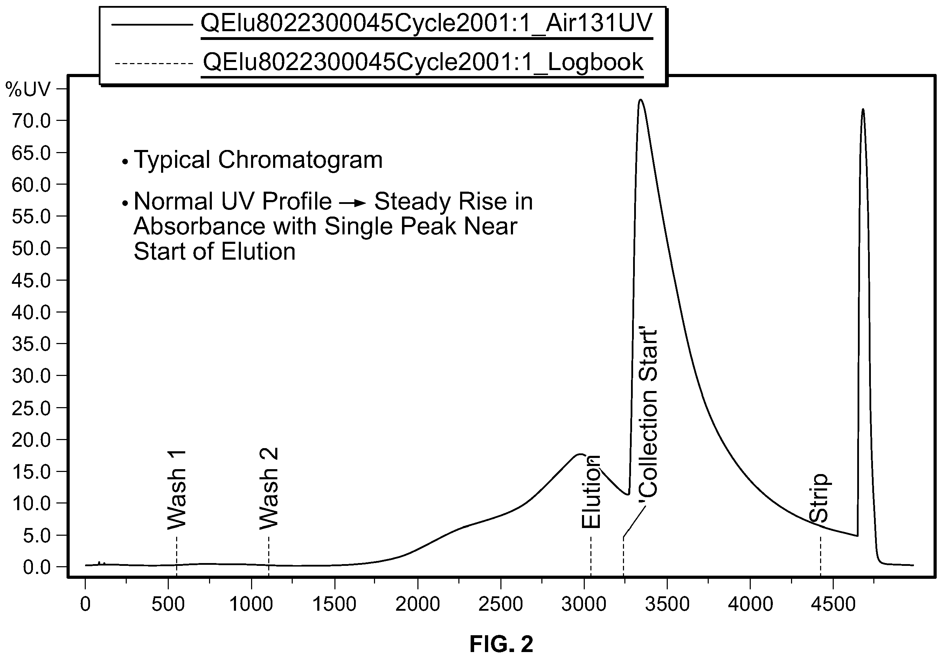

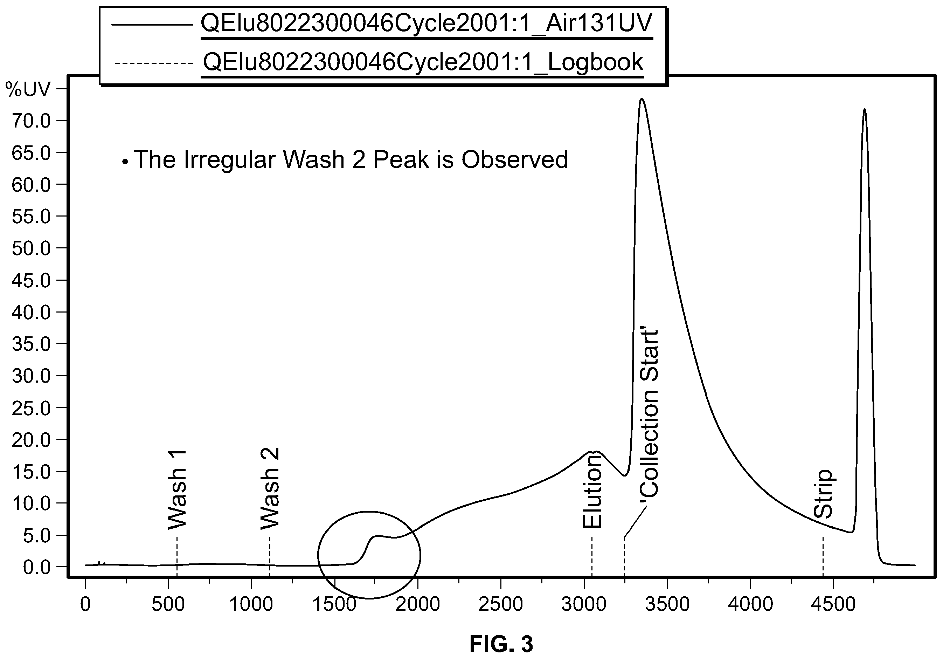

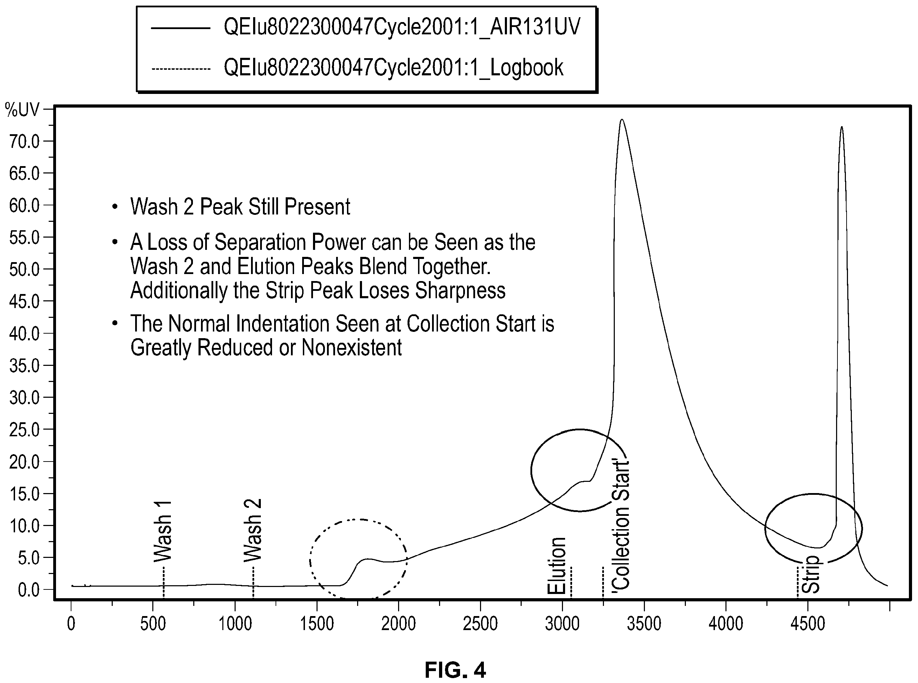

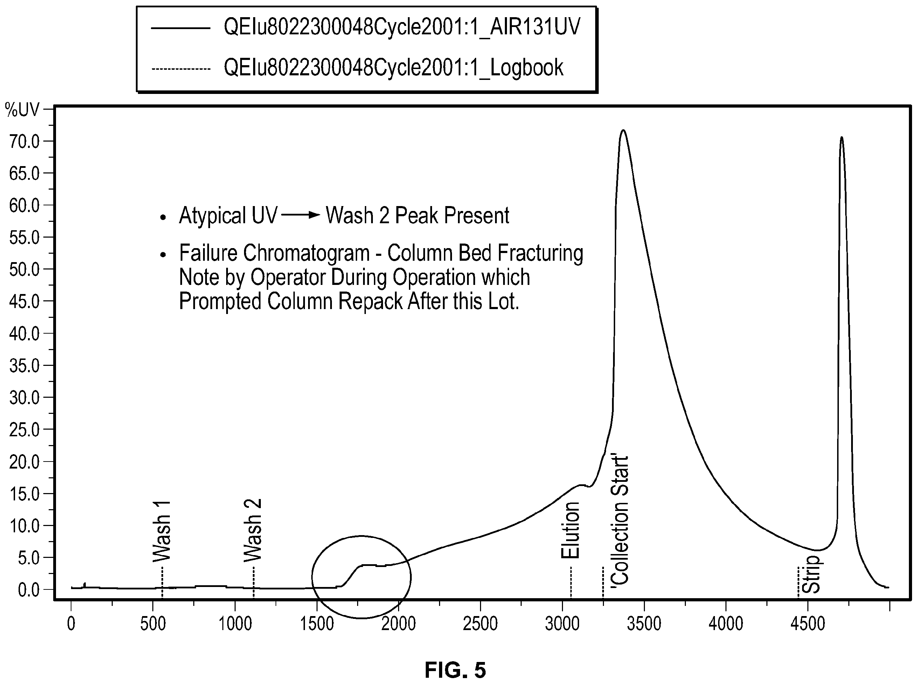

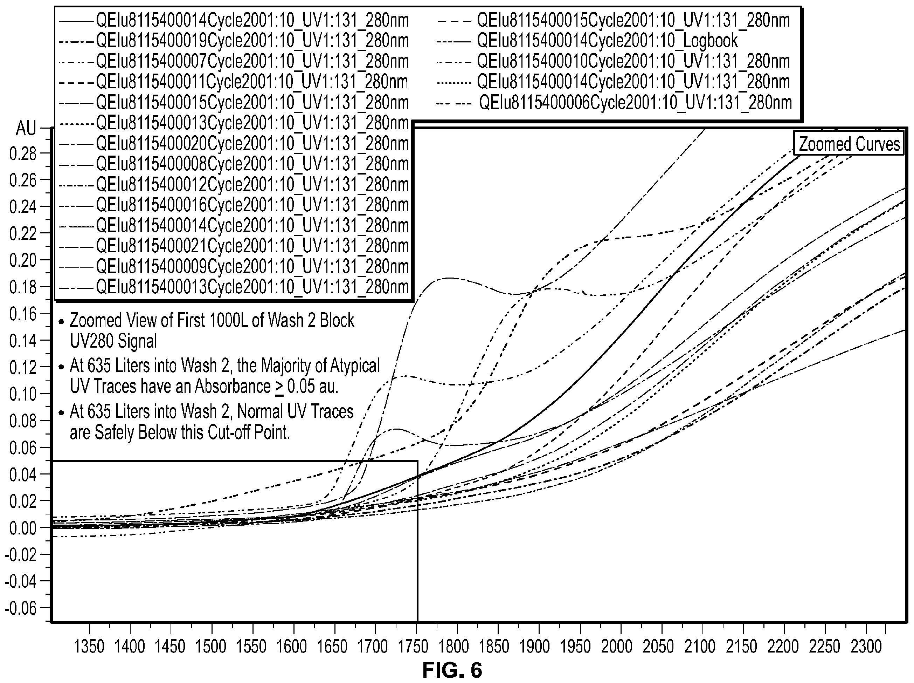

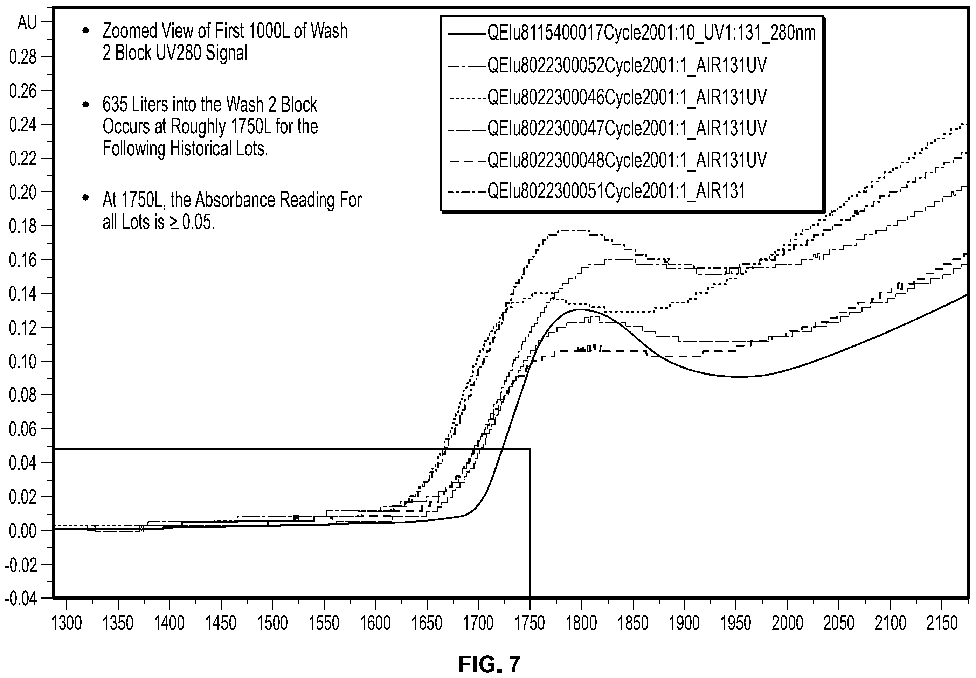

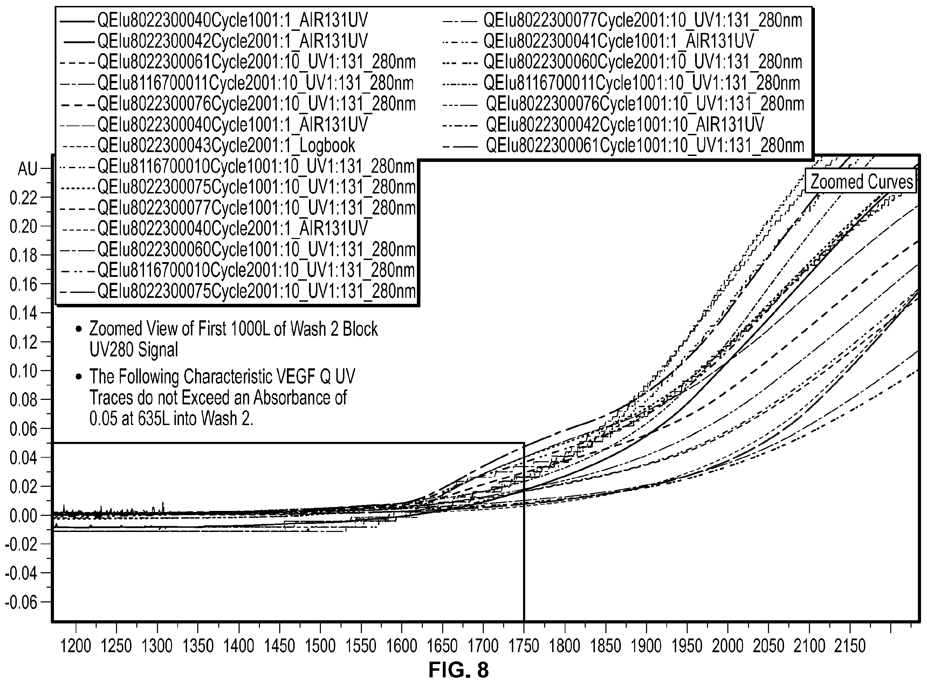

Using a VEGF-Trap as an exemplary analyte for purification, Figures 1-8 depict both exemplary reference chromatograms (Figures 1 and 2) as well as exemplary process chromatograms having an atypical profile (Figures 3 through 5).

Briefly, the chromatograms of Figures 1-8 where generated by contacting a starting composition comprising a plurality of analytes (VEGF-Trap analytes as well as a plurality of contaminants (e.g. host cell proteins, DNA and poorly sialiated proteins)) and a separation composition comprising a matrix (in this example, Q Sepharose), under conditions sufficient for at least one VEGF-Trap protein of the starting composition to reversibly bind to the matrix, wherein the chromatography column comprises the separation composition, to produce an analyte-bound column and a first waste composition. Next, the analyte-bound column and a first washing composition were contacted, wherein the VEGF-Trap protein of the starting composition remains bound to the matrix to produce a purified analyte-bound column and a second waste composition. The analyte-bound column and a second washing composition were contacted, wherein, in the reference chromatogram depicting a typical profile, the VEGF-Trap protein of the starting composition remains bound to the matrix to produce a purified analyte-bound column and a third waste composition. To generate the process chromatograph depicting an atypical profile, the analyte-bound column and a second washing composition were contacted, wherein, in the process chromatogram depicting an atypical profile, only the VEGF-Trap protein of the starting composition remains bound to the matrix to produce a purified analyte-bound column and a third waste composition, however, the third waste composition comprises a contaminant or a VEGF-Trap protein. In both the reference and process chromatograms, the purified analyte-bound column and an elution composition are contacted, wherein the at least one analyte of the starting composition is released from the matrix into the elution composition to produce an eluted analyte composition. In both the reference and process chromatograms, the eluted analyte composition is collected. Simultaneously with each of above, each of the first waste composition, the second waste composition, the third waste composition and the eluted analyte composition are contacted with ultraviolet (UV) light. Simultaneously or sequentially, an absorbance of the UV light of each of the first waste composition, the second waste composition, the third waste composition and the eluted analyte composition is measured to produce an absorbance value for each of the first waste composition, the second waste composition, the third waste composition, and the eluted analyte composition. The absorbance values for each of the first waste composition, the second waste composition, the third waste composition and the eluted analyte composition were plotted as a function of volume of liquid that traverses the chromatography column to generate a chromatogram. Generation of the chromatograph occurs in real-time with the chromatography process and the occurrence of an atypical profile is determined as the additional peak appears on the chromatogram. The appearance of an atypical profile causes a detector and/or a processor to send a warning signal to a processor operably-linked to the chromatography column, which, in turn, prevents a subsequent chromatography process until the malfunction of the column can be resolved.

## Claims

1. A method for detecting a malfunction in a chromatography process, comprising:
(a) generating a reference chromatogram from a chromatography column,
(b) generating a process chromatogram from the chromatography column, wherein the process chromatogram comprises a measured absorbance intensity, a maximum absorbance intensity, and an integral volume window,
(c) detecting an atypical profile in the process chromatogram,
wherein the atypical profile indicates a potential or actual malfunction of a chromatography process, and wherein the atypical profile comprises:
(i) an additional peak on the process chromatogram when compared to a comparable position on the reference chromatogram, or
(ii) a measured absorbance intensity that exceeds a maximum absorbance intensity during an integral volume window,
wherein the atypical profile occurs during a washing step in the chromatography process and wherein said washing step precedes an elution step in the chromatography process,
(d) generating a warning signal when the atypical profile is detected, and
(e) interrupting the chromatography process until the potential or actual malfunction of the chromatography column is resolved,
wherein the chromatography column is suitable for use in ion exchange chromatography, affinity chromatography or hydrophobic interaction chromatography.

2. The method of claim 1, wherein the method further comprises (f) repacking the chromatography column.

3. The method of claim 1 or claim 2, wherein the potential or actual malfunction of the chromatography process is caused by column deterioration,
or wherein the potential or actual malfunction of the chromatography process is caused by column deterioration and the column deterioration comprises column bed fracturing.

4. The method of claim 1, wherein:
the magnitude of the additional peak indicates a degree of severity of the potential or actual malfunction of the chromatography process; or
the magnitude of the additional peak indicates a degree of severity of the potential or actual malfunction of the chromatography process and an increase in the magnitude of the additional peak indicates an increase in the degree of severity of the potential or actual malfunction of the chromatography process.

5. The method of claim 4, wherein the malfunction of the chromatography process induces a loss of separation power of the chromatography column.

6. The method of claim 1, wherein:
the measured absorbance intensity is determined at a wavelength corresponding to ultraviolet (UV) light; or
the measured absorbance intensity is determined at a wavelength corresponding to ultraviolet (UV) light and the measured absorbance intensity is determined at a wavelength of:
(a) between 260 nanometers (nm) and 280 nm, inclusive of the endpoints; or
(b) 260 nm, providing an A₂₆₀ value; or
(c) 280 nm, providing an A₂₈₀ value; or
the measured absorbance intensity is determined at a wavelength corresponding to ultraviolet (UV) light and the measured absorbance intensity is determined at a wavelength of 260 nm, providing an A₂₆₀ value, or 280 nm, providing an A₂₈₀ value, and wherein the maximum absorbance intensity has an A₂₆₀ value or an A₂₈₀ value of at least 0.05.

7. The method of any one of claims 1-6, wherein a typical profile comprises a measured absorbance intensity that does not exceed a maximum absorbance intensity during an integral volume window and wherein the maximum absorbance intensity has an A₂₆₀ value or an A₂₈₀ value of 0.05 or less.

8. The method of any one of claims 1-7, wherein the integral volume window occurs:
(a) between 1000 L and 2500 L, inclusive of the endpoints; or
(b) between 1250 L and 2250 L, inclusive of the endpoints; or
(c) between 1600 L and 1800 L, inclusive of the endpoints.

9. The method of any one of claims 1-8, wherein the chromatography column comprises:
(a) a matrix, and the matrix comprises a resin; or
(b) a Q Sepharose anion exchange resin.

10. The method of any one of claims 1-9, wherein the step of (a) generating the reference chromatogram and/or the step of (b) generating the process chromatogram comprises
(1) contacting a starting composition comprising a plurality of pre-determined analytes and a separation composition comprising a matrix, under conditions sufficient for at least one analyte of the starting composition to reversibly bind to the matrix, wherein the chromatography column comprises the separation composition, to produce an analyte-bound column and a first waste composition;
(2) contacting the analyte-bound column of (1) and a washing composition, wherein only the at least one analyte of the starting composition remains bound to the matrix to produce a purified analyte-bound column and a second waste composition;
(3) contacting the purified analyte-bound column of (2) and an elution composition, wherein the at least one analyte of the starting composition is released from the matrix into the elution composition to produce an eluted analyte composition;
(4) collecting the eluted analyte composition;
(5) simultaneously with each of steps (1)-(4), contacting each of the first waste composition, the second waste composition, and the eluted analyte composition with ultraviolet (UV) light,
(6) measuring an absorbance of the UV light of each of the first waste composition, the second waste composition, and the eluted analyte composition to produce an absorbance value for each of the first waste composition, the second waste composition, and the eluted analyte composition; and
(7) generating a chromatogram by plotting the absorbance value of each of the first waste composition, the second waste composition, and the eluted analyte composition as a function of volume of liquid that traverses the chromatography column.

11. The method of claim 10, wherein the contacting step (5) and the measuring step (6) are continuous.

12. The method of claim 10 or claim 11, wherein:
the test analyte is a biologic composition; or
the test analyte is a biologic composition and the biologic composition is a therapeutic composition for use in a method of treatment of a human subject.

13. The method of claim 12, wherein the biologic composition comprises a VEGF-Trap protein.

14. The method of any one of claims 1-13, wherein:
the warning signal is communicated electronically to a processor and wherein the processor is operably linked to the chromatography column; or
the warning signal is communicated electronically to a processor and wherein the processor is operably linked to the chromatography column, and wherein the processor receives the warning signal and interrupts the chromatography process or prevents the initiation of a further chromatography process until the potential or actual malfunction of the chromatography column is resolved.

## Patentansprüche

1. Verfahren zum Erkennen einer Fehlfunktion in einem Chromatographieprozess, umfassend:
(a) Erzeugen eines Referenzchromatogramms aus einer Chromatographiesäule,
(b) Erzeugen eines Prozesschromatogramms aus der Chromatographiesäule, wobei das Prozesschromatogramm eine gemessene Extinktionsintensität, eine maximale Extinktionsintensität und ein Integral-Volumenfenster umfasst,
(c) Erkennen eines atypischen Profils im Prozesschromatogramm,
wobei das atypische Profil eine potenzielle oder tatsächliche Fehlfunktion eines Chromatographieprozesses anzeigt und wobei das atypische Profil Folgendes umfasst:
(i) einen zusätzlichen Peak auf dem Prozesschromatogramm im Vergleich zu einer vergleichbaren Position auf dem Referenzchromatogramm, oder
(ii) eine gemessene Extinktionsintensität, die eine maximale Extinktionsintensität während eines Integral-Volumenfensters überschreitet,
wobei das atypische Profil während eines Waschschritts in dem Chromatographieprozess auftritt und wobei der Waschschritt einem Elutionsschritt in dem Chromatographieprozess vorausgeht,
(d) Erzeugen eines Warnsignals, wenn das atypische Profil erkannt wird, und
(e) Unterbrechen des Chromatographieprozesses, bis die potenzielle oder tatsächliche Fehlfunktion der Chromatographiesäule behoben ist,
wobei die Chromatographiesäule zur Verwendung in der Ionenaustauschchromatographie, Affinitätschromatographie oder Hydrophob-Interaktions-Chromatographie geeignet ist.

2. Verfahren nach Anspruch 1, wobei das Verfahren ferner (f) das erneute Packen der Chromatographiesäule umfasst.

3. Verfahren nach Anspruch 1 oder Anspruch 2, wobei die potenzielle oder tatsächliche Fehlfunktion des Chromatographieprozesses durch Säulenverschlechterung verursacht wird,
oder wobei die potenzielle oder tatsächliche Fehlfunktion des Chromatographieprozesses durch Säulenverschlechterung verursacht wird und die Säulenverschlechterung Säulenbettfrakturierung umfasst.

4. Verfahren nach Anspruch 1, wobei:
die Größe des zusätzlichen Peaks einen Grad der Schwere der potentiellen oder tatsächlichen Fehlfunktion des Chromatographieprozesses anzeigt; oder
die Größe des zusätzlichen Peaks einen Schweregrad der potentiellen oder tatsächlichen Fehlfunktion des Chromatographieprozesses anzeigt und eine Erhöhung der Größe des zusätzlichen Peaks eine Erhöhung des Schweregrads der potentiellen oder tatsächlichen Fehlfunktion des Chromatographieprozesses anzeigt.

5. Verfahren nach Anspruch 4, wobei die Fehlfunktion des Chromatographieprozesses einen Verlust der Trennleistung der Chromatographiesäule induziert.

6. Verfahren nach Anspruch 1, wobei:
die gemessene Extinktionsintensität bei einer Wellenlänge bestimmt wird, die ultraviolettem (UV) Licht entspricht; oder
die gemessene Extinktionsintensität bei einer Wellenlänge bestimmt wird, die ultraviolettem (UV) Licht entspricht, und die gemessene Extinktionsintensität bestimmt wird bei einer Wellenlänge von:
(a) zwischen 260 Nanometer (nm) und 280 nm einschließlich der Endpunkte; oder
(b) 260 nm unter Bereitstellung eines A₂₆₀-Werts; oder
(c) 280 nm unter Bereitstellung eines A₂₈₀-Werts; oder die gemessene Extinktionsintensität bei einer Wellenlänge bestimmt wird, die ultraviolettem (UV) Licht entspricht, und die gemessene Extinktionsintensität bestimmt wird bei einer Wellenlänge von 260 nm, wodurch ein A₂₆₀-Wert bereitgestellt wird, oder 280 nm, wodurch ein A₂₈₀-Wert bereitgestellt wird, und wobei die maximale Extinktionsintensität einen A₂₆₀-Wert oder einen A₂₈₀-Wert von mindestens 0,05 aufweist.

7. Verfahren nach einem der Ansprüche 1-6, wobei ein typisches Profil eine gemessene Extinktionsintensität umfasst, die eine maximale Extinktionsintensität während eines Integral-Volumenfensters nicht überschreitet, und wobei die maximale Extinktionsintensität einen A₂₆₀-Wert oder einen A₂₈₀-Wert von 0,05 oder weniger aufweist.

8. Verfahren nach einem der Ansprüche 1-7, wobei das Integral-Volumenfenster auftritt:
(a) zwischen 1000 l und 2500 l einschließlich der Endpunkte; oder
(b) zwischen 1250 l und 2250 l, einschließlich der Endpunkte; oder
(c) zwischen 1600 l und 1800 l, einschließlich der Endpunkte.

9. Verfahren nach einem der Ansprüche 1-8, wobei die Chromatographiesäule Folgendes umfasst:
(a) eine Matrix, und wobei die Matrix ein Harz umfasst; oder
(b) ein Q-Sepharose-Anionenaustauscherharz.

10. Verfahren nach einem der Ansprüche 1-9, wobei der Schritt (a) des Erzeugens des Referenzchromatogramms und/oder der Schritt (b) des Erzeugens des Prozesschromatogramms umfasst
(1) Inkontaktbringen einer Startzusammensetzung, die mehrere vorbestimmte Analyten und eine Trennzusammensetzung, die eine Matrix umfasst, umfasst, unter Bedingungen, die ausreichen, dass mindestens ein Analyt der Startzusammensetzung reversibel an die Matrix bindet, wobei die Chromatographiesäule die Trennzusammensetzung umfasst, um eine analytgebundene Säule und eine erste Abfallzusammensetzung (Durchfluss) zu erzeugen;
(2) Inkontaktbringen der analytgebundenen Säule von (1) und einer Waschzusammensetzung, wobei nur der mindestens eine Analyt der Startzusammensetzung an die Matrix gebunden bleibt, um eine gereinigte analytgebundene Säule und eine zweite Abfallzusammensetzung zu erzeugen;
(3) Inkontaktbringen der gereinigten analytgebundenen Säule von (2) und einer Elutionszusammensetzung, wobei der mindestens eine Analyt der Startzusammensetzung aus der Matrix in die Elutionszusammensetzung freigesetzt wird, um eine eluierte Analytzusammensetzung zu erzeugen;
(4) Sammeln der eluierten Analytzusammensetzung;
(5) gleichzeitig mit jedem der Schritte (1)-(4), Inkontaktbringen von jeder der ersten Abfallzusammensetzung, der zweiten Abfallzusammensetzung und der eluierten Analytzusammensetzung mit ultraviolettem (UV) Licht,
(6) Messung einer Extinktion des UV-Lichts von jeder der ersten Abfallzusammensetzung, der zweiten Abfallzusammensetzung und der eluierten Analytzusammensetzung, um einen Extinktionswert für jede der ersten Abfallzusammensetzung, der zweiten Abfallzusammensetzung und der eluierten Analytzusammensetzung zu erzeugen; und
(7) Erzeugen eines Chromatogramms durch Auftragen des Extinktionswerts von jeder der ersten Abfallzusammensetzung, der zweiten Abfallzusammensetzung und der eluierten Analytzusammensetzung als Funktion des Volumens an Flüssigkeit, die die Chromatographiesäule durchquert.

11. Verfahren nach Anspruch 10, wobei der Schritt des Inkontaktbringens (5) und der Schritt der Messung (6) kontinuierlich erfolgen.

12. Verfahren nach Anspruch 10 oder Anspruch 11, wobei: der Testanalyt eine biologische Zusammensetzung ist; oder der Testanalyt eine biologische Zusammensetzung ist und die biologische Zusammensetzung eine therapeutische Zusammensetzung zur Verwendung in einem Verfahren zur Behandlung eines menschlichen Individuums ist.

13. Verfahren nach Anspruch 12, wobei die biologische Zusammensetzung ein VEGF-Trap-Protein umfasst.

14. Verfahren nach einem der Ansprüche 1-13, wobei:
das Warnsignal elektronisch an einen Prozessor übermittelt wird und wobei der Prozessor funktionsfähig mit der Chromatographiesäule verbunden ist; oder
das Warnsignal elektronisch an einen Prozessor übermittelt wird und wobei der Prozessor funktionsfähig mit der Chromatographiesäule verbunden ist, und wobei der Prozessor das Warnsignal empfängt und den Chromatographieprozess unterbricht oder die Initiierung eines weiteren Chromatographieprozesses verhindert, bis die potentielle oder tatsächliche Fehlfunktion der Chromatographiesäule behoben ist.

## Revendications

1. Procédé de détection d'un dysfonctionnement dans un processus de chromatographie comprenant :
(a) la génération d'un chromatogramme de référence à partir d'une colonne de chromatographie,
(b) la génération d'un chromatogramme de processus à partir de la colonne de chromatographie, le chromatogramme de processus comprenant une intensité d'absorbance mesurée, une intensité d'absorbance maximale, et une fenêtre volumique intégrale,
(c) la détection d'un profil atypique dans le chromatogramme de processus,
où le profil atypique indique un dysfonctionnement potentiel ou réel d'un processus de chromatographie, et où le profil atypique comprend :
(i) un pic supplémentaire sur le chromatogramme de processus comparé à une position comparable sur le chromatogramme de référence, ou
(ii) une intensité d'absorbance mesurée qui dépasse une intensité d'absorbance maximale pendant une fenêtre volumique intégrale,
dans lequel le profil atypique se produit pendant une étape de lavage dans le processus de chromatographie et dans lequel ladite étape de lavage précède une étape d'élution dans le processus de chromatographie,
(d) la génération d'un signal d'avertissement lorsque le profil atypique est détecté, et
(e) l'interruption du processus de chromatographie jusqu'à ce que le dysfonctionnement potentiel ou réel de la colonne de chromatographie soit résolu,
dans lequel la colonne de chromatographie est appropriée pour être utilisée dans la chromatographie d'échange d'ions, la chromatographie d'affinité ou la chromatographie d'interactions hydrophobes.

2. Procédé selon la revendication 1, dans lequel le procédé comprend en outre (f) le regarnissage de la colonne de chromatographie.

3. Procédé selon la revendication 1 ou la revendication 2, dans lequel le dysfonctionnement potentiel ou réel du processus de chromatographie est provoqué par une détérioration de la colonne,
ou dans lequel le dysfonctionnement potentiel ou réel du procédé de chromatographie est causé par la détérioration de la colonne et la détérioration de la colonne comprend une fracturation du lit de colonne.

4. Procédé selon la revendication 1, dans lequel :
l'amplitude du pic supplémentaire indique un degré de gravité du dysfonctionnement potentiel ou réel du processus de chromatographie ; ou
l'amplitude du pic supplémentaire indique un degré de gravité du dysfonctionnement potentiel ou réel du processus de chromatographie et une augmentation de l'amplitude du pic supplémentaire indique une augmentation du degré de gravité du dysfonctionnement potentiel ou réel du processus de chromatographie.

5. Procédé selon la revendication 4, dans lequel le dysfonctionnement du processus de chromatographie induit une perte de pouvoir de séparation de la colonne de chromatographie.

6. Procédé selon la revendication 1, dans lequel :
l'intensité d'absorbance mesurée est déterminée à une longueur d'onde correspondant à la lumière ultraviolette (UV) ; ou
l'intensité d'absorbance mesurée est déterminée à une longueur d'onde correspondant à la lumière ultraviolette (UV) et l'intensité d'absorbance mesurée est déterminée à une longueur d'onde de :
(a) entre 260 nanomètres (nm) et 280 nm, limites incluses ; ou
(b) 260 nm, fournissant une valeur A₂₆₀ ; ou
(c) 280 nm, fournissant une valeur A₂₈₀ ; ou l'intensité d'absorbance mesurée est déterminée à une longueur d'onde correspondant à la lumière ultraviolette (UV) et l'intensité d'absorbance mesurée est déterminée à une longueur d'onde de 260 nm, fournissant une valeur A₂₆₀, ou 280 nm, fournissant une valeur A₂₈₀, et l'intensité d'absorbance maximale ayant une valeur A₂₆₀ ou une valeur A₂₈₀ d'au moins 0,05.

7. Procédé selon l'une quelconque des revendications 1 à 6, dans lequel un profil typique comprend une intensité d'absorbance mesurée qui ne dépasse pas une intensité d'absorbance maximale pendant une fenêtre volumique intégrale et dans lequel l'intensité d'absorbance maximale a une valeur A₂₆₀ ou une valeur A₂₈₀ de 0,05 ou moins.

8. Procédé selon l'une quelconque des revendications 1 à 7, dans lequel la fenêtre volumique intégrale a lieu :
(a) entre 1 000 L et 2 500 L, bornes incluses ; ou
(b) entre 1 250 L et 2 250 L, bornes incluses ; ou
(c) entre 1 600 L et 1 800 L, bornes incluses.

9. Procédé selon l'une quelconque des revendications 1 à 8, dans lequel la colonne de chromatographie comprend :
(a) une matrice, et la matrice comprend une résine ; ou
(b) une résine échangeuse d'anions de Q Sépharose.

10. Procédé selon l'une quelconque des revendications 1 à 9, dans lequel l'étape de (a) génération du chromatogramme de référence et/ou l'étape de (b) génération du chromatogramme de processus comprend
(1) mise en contact d'une composition de départ comprenant une pluralité d'analytes prédéterminés et d'une composition de séparation comprenant une matrice, dans des conditions suffisantes pour qu'au moins un analyte de la composition de départ se lie de manière réversible à la matrice, la colonne de chromatographie comprenant la composition de séparation, pour produire une colonne liée à un analyte et une première composition de déchets ;
(2) mise en contact de la colonne liée à un analyte de (1) et d'une composition de lavage, seul l'au moins un analyte de la composition de départ restant lié à la matrice pour produire une colonne liée à un analyte purifiée et une seconde composition de déchets ;
(3) mise en contact de la colonne liée à un analyte purifiée de (2) et d'une composition d'élution, l'au moins un analyte de la composition de départ étant libéré de la matrice dans la composition d'élution pour produire une composition d'analyte éluée ;
(4) collecte de la composition d'analyte éluée ;
(5) simultanément à chacune des étapes (1)-(4), mise en contact de chacune de la première composition de déchets, de la seconde composition de déchets et de la composition d'analyte éluée avec de la lumière ultraviolette (UV),
(6) mesure de l'absorbance de la lumière UV de chacune de la première composition de déchets, de la seconde composition de déchets et de la composition d'analyte éluée pour produire une valeur d'absorbance pour chacune de la première composition de déchets, de la seconde composition de déchets et de la composition d'analyte éluée ; et
(7) génération d'un chromatogramme en traçant la valeur d'absorbance de chacune de la première composition de déchets, de la seconde composition de déchets et de la composition d'analyte éluée en fonction du volume de liquide qui traverse la colonne de chromatographie.

11. Procédé selon la revendication 10, dans lequel l'étape de mise en contact (5) et l'étape de mesure (6) sont continues.

12. Procédé selon la revendication 10 ou la revendication 11, dans lequel :
l'analyte d'essai est une composition biologique ; ou
l'analyte d'essai est une composition biologique et la composition biologique est une composition thérapeutique destinée à être utilisée dans un procédé de traitement d'un sujet humain.

13. Procédé selon la revendication 12, dans lequel la composition biologique comprend une protéine VEGF-Trap.

14. Procédé selon l'une quelconque des revendications 1 à 13, dans lequel :
le signal d'avertissement est communiqué électroniquement à un processeur et dans lequel le processeur est relié de manière fonctionnelle à la colonne de chromatographie ; ou
le signal d'avertissement est communiqué électroniquement à un processeur et dans lequel le processeur est relié de manière opérationnelle à la colonne de chromatographie, et dans lequel le processeur reçoit le signal d'avertissement et interrompt le processus de chromatographie ou empêche le lancement d'un autre processus de chromatographie jusqu'à ce que le dysfonctionnement potentiel ou réel de la colonne de chromatographie soit résolu.
